# EUROPEAN PATENT SPECIFICATION

(11) **EP 0 633 027 B1**
(45) Date of publication and mention of the grant of the patent: **24.10.2001**
(21) Application number: 93919734.9
(22) Date of filing: 09.08.1993
(51) Int. Cl.: A61K 33/14, A61K 41/00

(54) **COMPOUND FOR THE BIOLOGICAL STIMULATION AND CORRECTION OF THE PHENOTYPE OF AN ORGANISM AND A PROCESS FOR PRODUCING THE SAME**
VERBINDUNG ZUR BIOLOGISCHEN STIMULATION UND KORREKTUR DES PHENOTYPS EINES ORGANISMUS UND VERFAHREN FÜR IHRE HERSTELLUNG
COMPOSE DE STIMULATION ET DE CORRECTION BIOLOGIQUES DU PHENOTYPE D'UN ORGANISME ET SON PROCEDE DE PRODUCTION

(30) Priority: 30.10.1992 RU 9203081; 10.11.1992 RU 9205267; 15.02.1993 RU 9308264
(43) Date of publication of application: 11.01.1995
(73) Proprietor: Markov, Gennady Alexandrovich, Novosibirsk, 630055 (RU)
(72) Inventor: Markov, Gennady Alexandrovich, Novosibirsk, 630055 (RU)
(74) Representative: Lord, Hilton David
(86) International application number: RU9300189
(87) International publication number: WO9409797

(56) References cited:
- EP-A- 0 330 800
- EP-A- 0 407 804
- WO-A-86/00228
- WO-A-89/10746
- WO-A-92/18136
- DE-A- 3 028 084
- FR-A- 2 477 418
- FR-A- 2 640 510
- GB-A- 411 499
- US-A- 5 188 738
- DATABASE WPI Section Ch, Week 9206 Derwent Publications Ltd., London, GB; Class B04, AN 92-045614 XP002059211 & JP 03 290 170 A (NIPPON KAYAKU KK) , 19 December 1991

## Description

### Field of the Invention

The present invention relates to biology and medicine and more specifically to compositions for effecting the inheritable structure and for treating the animals and human being.

### Prior Art

Said composition may be widely used in the R & D as well as practical activity including to research the mutagenises of organisms and in practice for treating different diseases of animals and human being such as alopecia, oncological diseases of thyroid gland, gastrointestinal tract, kidneys and urinary organs, male and femals sex organs.

These compositions are supposed to meet a number of requirements. Said compositions must not produce any side effects but shall have a high density of information, be stored for a long time and preferably under room conditions as well as shall possess good taste properties.

There are known conventional medicinal preparations obtained under the effect of outside power source (see US, A, 4,771,130).

The conventional preparation comprises three chemically bonded components:
(1) therapeutically active medicinal component which exerts influence upon suitable sites of a receptor accessible to form covalent bonds;
(2) therapeutically acceptable component able to be activated under the effect of an outside power source (visible, ultraviolet or X-rays) to form a covalent bond of component(s) of a medicament with specific sites of a receptor;
(3) therapeutically acceptable component for bonding components 1 and 2.

Said conventional preparation is multicomponent, i.e. comprising actually three preparations and without influencing the inheritable structure of an organism.

There is a known wound healing agent (see SU Pat.A 1,553,134) comprising sodium alginate, antiseptic and calcium salt, consisting of two compositions wherein a first composition contains ingredients at a ratio thereof in wt. percent as follows:

| | |
|---|---|
| sodium salt | 12 to 14 |
| glycerine | 0.5 to 1.5 |
| antiseptic | 0.1 to 6.0 |
| water | balance |

a second composition contains ingredients at a ratio, in wt. percent as follows:

| | |
|---|---|
| CaCl2 | 1 to 2 |
| glycerine | 0.5 to 1.0 |
| water | balance |

Said conventional healing agent does not exert any influence upon the inheritable structure of an organism.

Known in the prior art are methods for effecting an organism with different factors. Thus, known are a method intensifying the metabolism and growth of microorganisms and a device for performing the same (see DE. Al OS 3,538,194). Said conventional method is characterized in that microorganisms are subjected to an impact of electric field whereby the field effect is in the form of E-pulse and does not exceed 3.5 kV/cm.

There is a known method for the electric stimulation of articular chondrocytes (see US A 4,487,834). Said conventional method is that chondrocytes are carefully subjected to an impact of the AC field by connecting a capacitor to the electric circuit. The electric field is characterized with frequency of 60 Hz and electric current density in a range of from 15 to 145 microA/cm2.

There is a known method for correcting a biological system (see DE Al OS 3,505,685). Said conventional method is characterized in that genetic corrections necessary for treating animal, human and vegetal biological systems are carried out by means of artificial mutations of chromosomes. The latter are induced by hydridizing the cellular walls by means of radiological effects such as ultra-violet, X-rays, radiations of electrons, nuclear inos, neutrons and neutrinoes. Hence a type of gene mutations becomes target oriented, selective or determined due to a type of radiations, radiation dose and hardness.

Known in the prior art are methods of treating different diseases by using various radiations.

Known in the prior art is a method of treating patients having cardial ischemia (see SU.Al 1,537,276). Said conventional method comprises steps of effecting the region of heat and kidneys with a travelling pulse magnetic field having frequency in a range of from 2 to 10 kHz, field intensity in a range of from 1 to 3 mTl/cycle wherein the intensity of traveling magnetic field has frequency of 20 to 25 kHz and the intensity of the pulse magnetic field in the region of clavicles has freguency of 10 to 15 kHz, in the region of side surfaces of the thorax with frequency of 15 to 20 kHz whereby the total time of treatment effect is 30 to 40 minutes one or two sessions a day.

Known in the prior art is a conventional method of treating patients having gastroduodenal ulcers by using electromagnetic radiation (see SU, Al, 1,519,712). The conventional method is that daily for 10 to 30 minutes for 4 to 7 days a patient is under effect of the electromagnetic field whereby an ulcer defect of up to 1 cm diameter is treated by a power flux density of 1 mWt/cm2 with frequency of 37 GHz at a distance of 0.5 cm ant the ulcer whose diameter is more than 2 cm are treated by a power flux density of 15 mWt/cm2 with frequency of 78 GHz. at a distance of 2 cm.

### Summary of the Invention

Since the term "phenotype" means a set of all the features and properties of an organism being formed in its individual development, shaped due to an interaction of inheritable properties of the organism such as genotype and habitat the term "correction of the phenotype" means steps of influencing the pathological c hanges (or deviations) obtained in the life activity (impact of environment) as well as diseases transferred by inheritance. Thus, the term "correction of the phenotype of an organism" means steps of influencing the physiological, anatomical, pathological and inheritable structures of the organism.

It is an object of the invention to create such a composition for the biological stimulation and correction of the phenotype of an organism, which would exert impact upon the inheritable structure of the orragism, would be used to treat a number of diseases inclusive some malignant tumors, be without any side effect upon the organism and influence only the organs and tissues of the organism, which should be treated or processed. A high information density in the composition and long time for storing the information thereinhas been a task to create this composition.

The assigned task is attained by claiming a composition treated under the effect of the outside power source, containing food products such as aqueous solution of table salt and sugar in an amount, g/l as follows:

| | |
|---|---|
| salt | 1 to 400 |
| sugar | 1 to 3,000 |

There is claimed a composition which contains aqueous solution of sea salt and sugar in an amount, g/l as follows:

| | |
|---|---|
| sea salt | 1 to 400 |
| sugar | 1 to 3,000 |

There is claimed a composition wherein additionally to said components honey is added in an amount of 1 to 6,000 g/l.

There is claimed a composition wherein additionally thereto alkaline or alkali earth metal chlorides or carbonates or sea salt or mixture thereof in an amount of 1 to 320 g/l.

The claimed composition in the form of an additive may contain potassium chloride or carbonate in an amount of 1 to 320 g/l.

The claimed composition may contains as an additive calcium chloride or carbonate in an amount of 1 to 80 g/l.

The claimed composition may contain as an additive magnesium chloride or carbonate in an amount of 1 to 5 g/l.

The claimed composition may contain as as additive sea salt in an amount of 1 to 320 g/l.

The claimed composition may contain as an additive a mixture of said compounds in an amount of 2 to 320 g/l.

The claimed composition may contain microelements in the form of copper, manganese, molybdenum, iron, cobalt and nickel chlorides or carbonate in an amount of 1 to 4 g/l.

The assigned task is attained also by claiming a method of treating the composition under the effect of outside power source such as treating the composition by electric current pulses whose length is 10-8 up to 1 second with a voltage rate of pulse rise in a range of from 1x10⁻⁸ 1x10¹³ V/s and on-off time ratio in a range of from 1 to 10⁴ when the intensity of the electric field between electrodes is in a range of from 10 V/cm to 9x10⁴ V/cm.

The electric current in the present method may be preconverted into magnetic, electromagnetic, light or acoustic pulses.

As shown above, the composition for the stimulation and correction of an organism comprises as a base such food products as salt, sugar and honey. The aqueous solution of these components treated as shown above is able to effect different organs or tissues of an organism. The experiments allow to state that water treated with electric pulses shaped by a definite algorithm in a mode disclosed in the description of the method, stores the information for a short time (from 10 to 100 minutes) so that water has a close structural order only. To increase the information storage time (in a range of from 1 hour to three months) there were selected special aqueous solutions of substances which are not toxic for the organism and allow to increase the viscosity of water, forming polymer structures by processing it with electric signals.

An amount of main substances in the composition has been tested within some intervals broad enough and in all the cases this composition has been suitable to attain the assigned task. The ratios between the components have been selected also in a range sufficiently wide. Honey in the composition allows to improve the taste qualities and to increase a density of the introduced information but honey not always may be added thereto since some patients have allergy for honey and in some cases an incompatibility to honey is observed. Be sure that the composition for the effect of treatment as well as the algorithm of its treatment in each concrete case are selected experimentally.

In some cases the table salt may be replaced with sea salt due a necessity to increase the activity of the reaction in the organism to the administered preparation and to enlarge its applications for treating a number of specific diseases.

An additive in the form of alkaline (potassium) or alkali earth (calcium or magnesium) metal chlorides or carbonates or sea salt or a mixture of said compounds as well as microelements in the form of chlorides or carbonates introduced into the composition allow to enlarge the effect upon the organism and an amount of diseases which may be treated with a positive result.

Be also sure to note that there were a large amount of substances added thereto and their ratios and that all they were selected by experiments and correspond to the limits specified in the claims.

The choice of said additives containing inorganic salts of enumerated metals relative to sodium has been conditioned by physical-chemical properties of the metals themselves whereby these properties to a great extent are revealed when these metals are not in water but in an aqueous solution of salt and sugar. In addition said metals perform an important role in the organisms in vivo and the adsorption from the claimed compositions occurs with an effect more useful than their adsorption from the medicinal preparations. In addition, one of the factors more to prove a necessity to add metals to the claimed composition is a pathology of organs and tissues when said metals are absent and the introduction of these metals by means of the present composition is optimum.

The quantitative content of substances in the composition is conditioned by reasons as follows: the upper limit of table salt and sea salt is defined by table salt solubility in water whereby the total content of table salt and additive is limited also due to this reason. The upper limit of additive (320 g/l) is 80 percent of the maximum content of table salt since further a deficit of sodium begins with a negative impact upon the organism. This upper limit (320 g/l) is applicable only for potassium chloride or carbonate or sea salt but for alkali earch metals (calcium and magnesium) said limit is already not suitable since some problems appear when these metakls are removed these metals from the organism. The above also relates to the microelements (heavy metals) and that is why the upoper limit in the composition for calcium is limited with 80 g/l, for magnesium - with 8 g/l and for microelements - with 4 g/l. The lower content of all the other substances and additives is limited with 1 g/l, i.e. content with which the composition may exert influence upon the organism but for the first course of treatment of a patient or administration of a medicament a large amount of the composition is required.

The amount of sugar and honey has been established by their solubility and any further increase of the content is not expedient since the cost of the composition increases as well.

Modes in the method of treating the composition have been selected also to be wide enough since they correspond to the tasks to be attained with a concrete composition. In some cases the electric current passes through current converters to convert it into magnetic, electric, light or acoustic pulses.

The method of treating the compositions with electric current is characterized with a number of parameters wherein the most important parameter is the rate of pulse rise. The term "rate of pulse rise" means a velocity of voltage pulse amplitude growth in time, in V/cm. The information level transmitted to the cell depends upon said parameter, i.e. whether the organism cells will receive the information at the inheritable or physiological level.

Pulse on-off time ratio being a dimensionless parameter resides in a ratio of the period length (sum of a pluse length plus a pause) to the pulse length. Despite a scanty information in the pause without it the information cannot be transmitted to the organism or cell since the latter are limited in time for a step of assimilating the received signals.

### Detailed Description of the Preferred Embodiment

The method of preparing a composition comprises the steps as follows. Components are loaded into a vessel for a selected composition (in any mixture) such as salt or sea salt, sugar, honey if necessary, and an additive in a selected amount, they are dissolved in 1 liter of water by stirring and placed into a bath with electrodes. Then the electrodes are supplied with electric current whose parameters are described in the claims and the composition is processed in said mode for 3 minutes.

The compositions thus obtained have been used for treating different organisms such as daphnes, infusorias "shoes", for treating the animals and human being whereby in some cases the organisms have been placed into the composition as it was in a case with daphnes and infusorias and in other cases as it was in a case of treating the diseases the compositions have been administered orally or for the outside usage by smearing.

For providing the best understanding of the present invention there are recited concrete examples combined in two tables whereby Table 1 specifies compositions and Table 2 discloses the modes for treating them but the further usage of compositions will be described below.

**Table 1**

| Composition Quantitative Content of Ingredients | | | | | |
|---|---|---|---|---|---|
| No. | Salt g/l | Sea salt g/l | Suger g/l | Honey g/l | Additive (Composition and amount), g/l |
| 1 | 1 | - | 1 | - | - |
| 2 | 20 | - | 200 | 400 | - |
| 3 | 80 | 160 | 3000 | - | KCl-160 |
| 4 | - | 176 | 3000 | - | K2C03-176 |
| | | | | | CaCl2-40 |
| | | | | | MgCl2-8 |
| 5 | 192 | 80 | 3000 | | KCl-100 |
| | | | | | CaCO3-20 |
| | | | | | MgCO3-8 |
| 6 | 120 | 280 | 3000 | - | - |
| 7 | - | 5 | 3000 | - | KCl-160 |
| 8 | 200 | - | 2000 | - | KCl-40 |
| | | | | | CaCl2-1 |
| | | | | | NlCl2-1 |
| 9 | - | 200 | 3000 | - | KCl-40 |
| 10 | 320 | - | 3000 | - | CaCO3-80 |
| 11 | 390 | - | 3000 | 6000 | MgCl2-8 |
| 12 | 320 | - | 80 | 3000 | - |
| 13 | 80 | 200 | 3000 | - | K2CO3-112 |
| | | | | | MgCl2-8 |
| 14 | 76 | 160 | 3000 | - | KCl-116 |
| | | | | | CaCl2-40 |
| | | | | | MgCO3-4 |
| 15 | 200 | 180 | 3000 | - | KCl-20 |

**Table 2**

| Parameters for Treating the Compositions | | | | | | |
|---|---|---|---|---|---|---|
| No. | Length of pulses | Rate of pulse rise V/s | On-Off time ratio | Intensity, V/cm | Type of effect, | Voltage across a converter, kV |
| 1 | 1x10-8 | 1x10-8 | 1 | 9x10 4 | Electric current | - |
| 2 | 6x10-8 | 1x10 8 | 2 | - | Electro magnetic field | 20 |
| 3 | 1x10-8 | 1x10 8 | 2 | 9x10 4 | Electric field | - |
| | 2x10-8 | 1x10 7 | 7 | 8x10 4 | Idem | |
| | 3x10-7 | 8x10 6 | 18 | 7.3x10 4 | Idem | - |
| 4 | 4x10-8 | 9x10 6 | 4 | - | Idem | 20 |
| 5 | 1x10-4 | 1x10 8 | 20 | - | Light flux | 17 |
| 6 | 1x10-5 | 1x10 8 | 1x10 3 | - | Acoustic waves | 30 |
| 7 | 1x10-3 | 1x10 7 | 1x10 3 | 1x10 4 | Electric current | - |
| 8 | 1x10-1 | 8x10 4 | 2x10 2 | 1x10 3 | Idem | - |
| 9 | 1x10-1 | 4x10 3 | 7x10 2 | 1x10 2 | Idem | - |
| 10 | 1x10-2 | 1x10 3 | 18 | 10 | Idem | - |
| 11 | 2x10-2 | 3x10 5 | 56 | 1x10 3 | Idem | - |
| 12 | 4x10-3 | 1x10 6 | 8 | 1x10 3 | Idem | - |
| 13 | 7x10-5 | 1x10 8 | 2 | 3x10 3 | Idem | - |
| 14 | 3x10-2 | 8x10 2 | 80 | 8x10 2 | Idem | - |
| 15 | 6x10-3 | 2x10 5 | 32 | 1.3x10 3 | Idem | - |

### Example 1

Infusorias "shoes" taken from the same clone were placed into two vessels (working and test vessels) with pure water. Water in the working vessel was treated by using electric current in a mode as follows: pulse length being 1x10-8 s, rate of voltage pulse rise being 1x10-8 V/s, on-off time ratio being 1, electric field intensity between the bath electrodes being 100 kw/cm. As a result of the experiment in 10 minutes the full mitosis of cells was observed in the working vessel while there were no changes in the check vessel.

### Example 2

Infusorias "shoes" were placed into a vessel with non treated composition 1 to be therein for 4 hours with any change, after that they were removed from the vessel and the solution was treated by using the electric current in a mode specified. 30 minutes later infusorias "shoes" were placed into the vessel, the full division occurred for 20 seconds. When 10 days infusorias were placed into the solution it took 2 minutes for the full mitosis but when 20 days later they were placed therein the full mitosis was not observed. Thus, the composition not treated by the discribed method does not exert any impact upon a cell. The treated composition exerted effect thereupon for 10 days.

### Example 3

Daphnes were placed into a vessel containing a 3 percent acetone aqueous solution and they were kept therein practically to their full death (convulsive jerk of antennae) wherein the death of daphnes in this solution occurs in 4 hours. However 30 minutes after the composition 3 in an amount of 0.25 ml had been introduced into water the daphnesm reanimated and began swimming their full rehabilitation took place.

### Example 4

Infusorias "shoes" taken from the same clone were put into two vessels (working and test vessels). 0.1 ml of composition 7 containing the information about the complete destruction of the cell. There was observed a formation of two gas bubbles whose size grew continuously until the cell of the infusoria "shoe" was destroyed completely. Composition 7 not treated by said modes was added in an amount of 0.1 ml into the test vessel whereby the cells of infusorias "shoes" preserved the ability to the mitosis.

### Example 5

The sugar solution of food yeast was exposed for 14 days, and thereafter the fermentation was suspended. Then sugar was added to the solution in an amount of 50 percent of the original value. However the fermentation was not resumed since the yeast was without any vital power. Composition 14 was added to the solution in an amount of 50 ml (for one liter of yeast solution). Yeast began functioning and the fermentation continued until sugar was consumed completely.

### Example 6

Wheat grains for 6 hours were wetted in a 10 percent solution of composition 14 and the test grains at the same time were wetted for 6 hous in pure water. After that the grains were sown in the earth for the purpose of growth. The obtained results allow to state that the germination rate of grains increases from 10 percent to 80 percent and the stalk growth rate increases by 1.9 times as compared to the test field.

### Example 7

Composition 2 was tested by treating a sick dog (German herdsdog), 1 year old, which was ill of alopecia. Baldness began on the body of the sick dog, spots of 100 to 200 sq.cm appeared, the skin of the dog became blue. After a week course of treatment by using the composition by smearing the blue colour of sports disappeared , the skin became rosy. A week later the underwool of 1 cm high emerged.In three weeks the hair on the body was restored completely.

### Example 8

Patient D (for the city of Nizhnewartovsk), 10 years old, with a diagnosis of the spinal cord tumor for three years was treated in the oncological centers of Moscow and Leningrad. Despite the treatment the state of the patient sharply became worse since the tumor took the spinal cord canal from the second vertebra to the seventeenth one with an enlarged bore of the canal, the patient stopped holding the head, the speech disappeared, the initial stage of the paralization of organs and extrimites emerged. The patient was transferred to the symptomatic treatment and after that the parents of the patient applied for a course of treatment by using the claimed compositions. For treating the patient the composition 6 was used. For 10 days of the treatment course 80 ml of the claimed composition were administered to the patient. Then 10 days later a second course of treatment began by using the composition 4, and during the second course of treatment (10 days) 80 ml of composition were administered to the patient. After that the dynamics was positive, the gastrointestinal tract organs became normal, the speech was restored, and the paralysis of extrimities and organs disappeared. At present the treatment continues.

### Example 9.

Patient L. (from the city of Novorossiysk), 14 years old, with a diagnosis of the brain cancer for three years was treated in the oncological centers of Novorossiysk and Moscow. The cranial trepanation was carried out and a hole was made in the temporal part of the sculp for the tumor outlet. The illness progredded, the complete loss of vision, audition, speech, memory and full paralization of extrimities began. After the patient had been transferred to the symptomatic treatment the parent of the patient applied for a course of treatment by using the claimed compositions. For treating the disease use was made of composition 9 in an amount of 300 ml for 15 days, then after the interval (10 days) of composition 5 in an amount of 400 ml for 20 days. The state of the patient for the time of treatment improved. The vision was restored almost completely. The speech, memory, audition were restored, the paralysis of hands disappeared (the patient could take by hands small objects and by hands he could turn himself in the bed), a partial disppearance of the paralysis of feet was observed (he could stir feet), bedsores and decubituses disppeared. The treatment continues by using the novel compositions.

### Example 10

Patient N (from the city of Novosibirsk), 35 years old with a diagnosis of the uterus cancer was treated for two years in the Novosibirsk oncological center, took a course of treatment by radiation and three courses of chemical therapy. However the disease progressed, a metatatic spreading appeared in the right ovary and the hemorrhage was observed. The patient was transferred to the symptomatic treatment and after that the patient applied for a course of treatment with claimed compositions. For the treatment use was made of composition 10 as follows: 40 ml for the first course for 20 days. After that the hemorrhage stopped completely. The patient was transferred to a remission. After the second course of treatment by usining 400 ml of the same composition for 20 days the uterus tumor decreased by 1.5 times. At present the treatment continues.

### Example 11

Patient S. (from the city of Novosibirsk), 28 years old had a diagnosis of alopecia, from the time when she was 7 years old the acomia of hair began (the skin on the head is thin). By the age of 20 years the acomia on the head and body was complete. The patient was treated in the hospitals of Yerevan, Moscow and Kiev. The treatment did not produce any positive results. The patient was transferred to a symptomatic treatment. By the age of 25 year the skin atrophy began. She could not wear any wool clothes. The skin in winter was cracked due to a deficiency of moisture, The patient always used ointments made in China. When she was 28 years old, she applied for a course of treatment by using the claimed compositions. After the first course of treatment by composition 12 any reaction of the organism was not observed but a "fluff" appeared on the face but later it disappered.During the second course of treatment by using the composition 6 the disease was aggravated (the skin began itching, water bubbles appeared, pores on the head were inflamated, small absecesses appeared). By the end of the second course of treatment the aggravation began disappearing, the skin on the body restored its normal properties, a need to use the ointment for the body, the more dense "fluff" appeared on the face than after the first course of treatment. During the third course of treatment by using the composition 11 the skin was regenerated completely, it became soft and elastic, an irritation by wearing the wool clothers disappeared. The skin on the head acquired a normal colour, becoming more dense and thick and the pores of skin were restored. During the third course of treatment the hair appeared on the face, body, the destroyed enemal on the teeth was restored, a portion of the broken tooth was regenerated. After the fourth course of treatment by using the composition 13 the general state of the patient improved a great deal, the vital tone stepped up and the general area with hair increased. For each course of treatment the patient received by 400 ml of composition and the length of each course was 12 days with an interval between the courses was one month.

### Example 12

Patient K.(from the city of Yaroslavl), 10 years old, with a diagnosis of lymphoblastic leukosis was treated in the Yaroslavl oncological center. As a result he was conducted to a stage of remission with blastic cells present in the bone marrow in an amount of up to 5 percent. 4 months later the parents of the patient applied for a course of treatment by using the claimed compositions. For treating the disease composition 8 was used in an amount of 300 ml. During the treatment (for 22 days) the formula of blood changed, by the end of the treatment the bone marrow was regenerated with outlet of blastic cells into the blood in an amount of 12 percent and after that blastic cells in blood died and in the further analysis they were not detected. The analysis of the bone marrow confirmed the absence of blastic cells in the organism. The dynamics of the formula of blood during the treatment may be seen in Table 3.

**Table 3**

| Dynamics of the Blood Formula of the Patient (August, 1992) | | | | | | | | | |
|---|---|---|---|---|---|---|---|---|---|
| Description | 6 | 11 | 14 | 17 | 19 | 21 | 24 | 26 | 28 |
| Hemoglobins | 130 | 134 | 148 | 152 | 142 | 143 | 148 | 146 | 149 |
| Erythrocytes | 4.4 | 4.7 | 4.5 | 5.3 | 4.5 | 4.4 | 4.55 | 4.45 | 4.6 |
| | | | | | | | | | |
| Colour factor | 0.9 | 0.88 | 0.9 | 0.88 | 0.9 | 0.9 | 0.9 | 0.9 | 0.9 |
| | | | | | | | | | |
| Reticulocytes | | | | | 0.3 | | 0.4 | 0.5 | 0.6 |
| Thrombocytes | | | | | 210 | | 337 | 340 | 330 |
| Leucocytes | 2 | 3.8 | 2.7 | 1.8 | 2.3 | 2.2 | 1.2 | 2.2 | 2.6 |
| | | | | | | | | | |
| Stab neut-rophile | 3 | 4 | 1 | 2 | 0.25 | 2.5 | 3 | 2 | 3 |
| Segment | 37 | 24 | 28 | 15 | 12.5 | 7.5 | 29 | 39 | 48 |
| | | | | | | | | | |
| Eosino- philse | | 4 | 1 | 2 | | 1.5 | | 2 | 2 |
| Basophiles | 6 | | | 5 | | 0.5 | | | 1 |
| Limphocytes | 51 | 62 | 65 | 71 | 72 | 70 | 62 | 55 | 42 |
| Monocytes | 3 | 5.5 | 5 | 5.5 | 5 | 4.5 | 4 | 2 | 4 |
| SOE | 8 | 2 | 3 | 6 | 2 | 2 | 10 | 10 | 8 |
| | | | | | | | | | |
| Plasmatic cl. | | 0.5 | | | 1 | | 1 | 1 | 1 |
| | | | | | | | | | |
| Promyelocy-tes | | | | | 4 | | | | |
| Blasts | | | | | | 12.5 | | | |
| | | | | | | | | | |
| Polympho- cytes | | | | 5 | | | | | |
| Myelocytes | | | | | | 1 | | 1 | |
| Note: A space in the line means that an analysis result is absent or a component is absent | | | | | | | | | |

### Example 13

Patient D. (from the city of Novosibirsk), 31 years with a diagnosis of the acute lymphoblastic leukosis with the quick development of the disease passes three courses of treatment of chemical therapy in the ocnological center of Moscow in the hemitological Department of the Military Hospital. 6 months after the treatment the patient was at the stage of remission for 6 months and after a new recurrence he applied for a course of treatment by using the claimed compositions. He passed quickly one course of treatment by using the composition 15 for one week. The analysis results shown the braking of the disease development. The results of the dynamics in the blood formula are shown in Table 4.

**Table 4**

| Dynamics of the Blood Formula of the Patent (August to September, 1992) | | | | | | |
|---|---|---|---|---|---|---|
| Description | 22 | 29 | 30 | 1 | 2 | 5 |
| Hemoglobin | 171 | 160 | 168 | 163 | 166 | 155 |
| Erythrocytes | 5.6 | 5.4 | 5.6 | 5 | 5.2 | 4.9 |
| Colour factor | 0.9 | | | 0.9 | 0.9 | 0.9 |
| Reticulocytes | | 2.4 | 2.4 | 2 | 2.7 | |
| Thrombocytes | 200 | 280 | 240 | 240 | 260 | 220 |
| Leucocytes | 10.5 | 11 | 12.3 | 10.1 | 9.7 | 9 |
| Stab neutrophiles | 1.5 | 2 | 2.5 | 3.5 | 1 | 6 |
| Segment | 42 | 40 | 41 | 37.5 | 35 | 42.5 |
| Eosinophiles | 2 2 | 0.5 | | 2 | 2 | 2 |
| Basophiles | 1 | | | 1 | 0.5 | 0.5 |
| Lymphocytes | 48 | 44 | 32.5 | 22 | 35 | 40 |
| Monocytes | 2.5 | 1.5 | 1 | 1 | 1 | 3 |
| SOE | 4 2 | 2 | 3 | 2 | 3 | 19 |
| Myelocytes | 2 2 | 2 | 1 | 1.5 | 0.5 | |
| Blasts | 1 10 | 10 | 21 | 31.5 | 27 | 5.0 |
| Note: A space in the line means that an analysis result is absent or a component is absent | | | | | | |

Thus, two last examples shows that the claimed compositions may be used for treating the blood leukosis.

### Industrial Applicability

The proposed composition may be widely used in the R & D and practical activity in biology, medicine and veterinary.

In biology the proposed composition may be used for mutagenesis of organisms, for changing their phenotype and inheritable structure. Steps of treating the seeds by this composition prior to sowing them allows to increase the harvest rate and resistance thereof to different type of diseases.

In veterinary the composition may be used for treating the animals in particular, such as alopecia. In medicine the composition may be used for treating the diseases of a human being, such as alopecia, oncological diseases of tyroid gland, gastrointestinal tract, kidneys and urinary ways, male and female sex organs.

The proposed composition may be used also for the correction and stimulation of the human organism, in particular, old people.

The proposed composition contains food products and some inorganic additives which enter the salt composition of the blood of the organism and that is why said composition does not exert any harmful effect upon the organism and due to the wide range of additives it may be used also for treating such a disease as diabetes.

Due to the treatment of the composition by using different type of radiations it preserves the information introduced thereinto for a long time, may be stored under conventional conditions without chaning a quantitative ratio of substances and without any decomposition.

## Claims

1. A composition for biological stimulation and correction of phenotype of the organism comprising table salt and sugar treated under the effect of an outside power source, **characterised in that** it comprises water solution of table salt and sugar in the following amounts, g/l: salt 1-400, sugar 1-3000 and treated with electric current of pulse duration from 1 x 10⁻⁸ to 1 second with a rate of voltage pulse rise in a range of from 1 x 10⁻⁸ - 1 x 10¹³ V/s and on-off time ratio of from 1 to 10⁴ when the electric field intensity between electrodes is in a range of from 10 to 9 x 10⁴ V/cm.

2. A composition for biological stimulation and correction of phenotype of the organism comprising sea salt and sugar treated under the effect of an outside power source, **characterised in that** it comprises water solution of sea salt and sugar in the following amounts, g/l: salt 1 - 400, sugar 1-3000 and treated with electric current of pulse duration from 1 x 10⁻⁸ to 1 second with a rate of voltage pulse rise in a range of from 1 x 10⁻⁸ - 1 x 10¹³ V/s and on-off time ratio of from 1 to 10⁴ when the electric field intensity between electrodes is in a range of from 10 to 9 x 10⁴ V/cm.

3. A composition according to Claims 1 and 2, **characterised in that** said composition additionally contains honey in an amount of 1 to 6000 g/l.

4. A composition according to Claims 1-3, **characterised in that** said composition additionally contains an additive of alkaline or alkali-earth chlorides or carbonate or sea salt or a mixture of these compounds in an amount of 1 to 320 g/l.

5. A composition according to Claims 1-4, **characterised in that** as an additive use is made of potassium chloride or carbonate in an amount of 1 to 320 g/l.

6. A composition according to Claims 1-4, **characterised in that** as an additive-use is made of calcium chloride or carbonate in an amount of 1 to 80 g/l.

7. A composition according to Claims 1-4, **characterised in that** as an additive use is made of magnesium chloride or carbonate in an amount of I to 5 g/l.

8. A composition according to Claims 1, 3 and 4, **characterised in that** as an additive use is made of sea salt in an amount of 1 to 320 g/l.

9. A composition according to Claims 4, **characterised in that** as an additive use is made of a mixture of said compounds in an amount of 2 to 320 g/l.

10. A composition according to Claims 1-9, **characterised in that** said composition additionally contains microelements in the form of copper, molybdenum, manganese, iron, cobalt, nickel chlorides or carbonates in an amount of 1 to 4 g/l.

11. A composition according to Claims 1 and 2, **characterised in that** electric pulses are preconverted into magnetic, electromagnetic, light or acoustic pulses.

## Patentansprüche

1. Zusammensetzung für die biologische Stimulation und Korrektur des Phänotyps des Lebewesens, aufweisend Tafelsalz und Zucker, behandelt unter der Einwirkung einer äußeren Energiequelle, **dadurch gekennzeichnet, dass** sie eine wässrige Lösung von Tafelsalz und Zucker in den folgenden Mengen in Gramm pro Liter aufweist: 1 bis 400 Salz, 1 bis 3.000 Zucker; und dass sie behandelt wird mit elektrischem Strom einer Impulsdauer von 1x10⁻⁸ bis 1 Sekunden mit einer Rate des Spannungspulsanstieges im Bereich von 1x10⁻⁸ bis 1x10¹³ V/s und mit einem Ein-/Ausschaltzeitverhältnis von 1 bis 10⁴, wenn die elektrische Feldstärke zwischen den Elektroden im Bereich von 10 bis 9x10⁴ V/cm liegt.

2. Zusammensetzung für die biologische Stimulation und Korrektur des Phänotyps des Lebewesens, aufweisend Meeressalz und Zucker, behandelt unter der Einwirkung einer äußeren Energiequelle, **dadurch gekennzeichnet, dass** sie eine wässrige Lösung von Meeressalz und Zucker in den folgenden Mengen in Gramm pro Liter aufweist: 1 bis 400 Salz, 1 bis 3.000 Zucker; und dass sie behandelt wird mit elektrischem Strom einer Impulsdauer von 1x10⁻⁸ bis 1 Sekunden mit einer Rate des Spannungspulsanstieges im Bereich von 1x10⁻⁸ bis 1x10¹³ V/s und mit einem Ein-/Ausschaltzeitverhältnis von 1 bis 10⁴, wenn die elektrische Feldstärke zwischen den Elektroden im Bereich von 10 bis 9x10⁴ V/cm liegt.

3. Zusammensetzung nach Anspruch 1 und 2, **dadurch gekennzeichnet, dass** die Zusammensetzung zusätzlich Honig in einer Menge von 1 bis 6.000 g/l enthält.

4. Zusammensetzung nach Anspruch 1 bis 3, **dadurch gekennzeichnet, dass** die Zusammensetzung zusätzlich ein Additiv von Alkali- oder Erdalkalichloriden oder -carbonaten oder Meeressalz oder eine Mischung diese Verbindungen in einer Menge von 1 bis 320 g/l enthält.

5. Zusammensetzung nach Anspruch 1 bis 4, **dadurch gekennzeichnet, dass** als ein Additiv Kaliumchlorid oder -carbonat in einer Menge von 1 bis 320 g/l verwendet wird.

6. Zusammensetzung nach Anspruch 1 bis 4, **dadurch gekennzeichnet, dass** als ein Additiv Calciumchlorid oder -carbonat in einer Menge von 1 bis 80 g/l verwendet wird.

7. Zusammensetzung nach Anspruch 1 bis 4, **dadurch gekennzeichnet, dass** als ein Additiv Magnesiumchlorid oder -carbonat in einer Menge von 1 bis 5 g/l verwendet wird.

8. Zusammensetzung nach Anspruch 1, 3, 4, **dadurch gekennzeichnet, dass** als ein Additiv Meeressalz in einer Menge von 1 bis 320 g/l verwendet wird.

9. Zusammensetzung nach Anspruch 1 bis 4, **dadurch gekennzeichnet, dass** als ein Additiv ein Mischung der Verbindungen in einer Menge von 2 bis 320 g/l verwendet wird.

10. Zusammensetzung nach Anspruch 1 bis 9, **dadurch gekennzeichnet, dass** die Zusammensetzung zusätzlich Mikroelemente in Form von Kupfer-, Mölybdän-, Mangan-, Eisen-, Kobalt-, Nickelchloriden oder -carbonaten in einer Menge von 1 bis 4 g/l enthält.

11. Zusammensetzung nach Anspruch 1 und 2, **dadurch gekennzeichnet, dass** elektrische Impulse in magnetische, elektromagnetische, Licht- oder akustische Impulse vorumgewandelt werden.

## Revendications

1. Composition pour la stimulation et la correction biologiques du phénotype de l'organisme comprenant du sel de table et du sucre, traitée sous l'effet d'une source d'énergie externe, **caractérisée en ce qu'**elle comprend une solution aqueuse de sel de table et de sucre dans les quantités suivantes, g/l : sel 1-400, sucre 1-3000 et traitée avec un courant électrique d'une durée d'impulsion de 1x10⁻⁸ à 1 seconde avec une vitesse d'augmentation des impulsions électriques dans la gamme de 1x10⁻⁸ à 1x10¹³ V/s et un ratio du temps de marche-arrêt de 1 à 10⁴ lorsque l'intensité du champ électrique entre les électrodes est dans la gamme de 10 à 9x10⁴ V/cm.

2. Composition pour la stimulation et la correction biologiques du phénotype de l'organisme comprenant du sel de mer et du sucre, traitée sous l'effet d'une source d'énergie externe, **caractérisée en ce qu'**elle comprend une solution aqueuse de sel de mer et de sucre dans les quantités suivantes, g/l : sel 1-400, sucre 1-3000 et traitée avec un courant électrique d'une durée d'impulsion de 1x10⁻⁸ à 1 seconde avec une vitesse d'augmentation des impulsions électriques dans la gamme de 1x10⁻⁸ à 1x10¹³ V/s et un ratio du temps de marche-arrêt de 1 à 10⁴ lorsque l'intensité du champ électrique entre les électrodes est dans la gamme de 10 à 9x10⁴ V/cm.

3. Composition selon les revendications 1 et 2, **caractérisée en ce que** ladite composition contient de plus du miel en une quantité de 1 à 6000 g/l.

4. Composition selon les revendications 1 à 3, **caractérisée en ce que** ladite composition contient de plus un additif de chlorures ou carbonates alcalins ou alcalinoterreux ou du sel de mer ou un mélange de ces composés en une quantité de 1 à 320 g/l.

5. Composition selon les revendications 1 à 4, **caractérisée en ce qu'**en tant qu'additif, on utilise du chlorure ou carbonate de potassium en une quantité de 1 à 320 g/l.

6. Composition selon les revendications 1 à 4, **caractérisée en ce qu'**en tant qu'additif, on utilise du chlorure ou carbonate de calcium en une quantité de 1 à 80 g/l.

7. Composition selon les revendications 1 à 4, **caractérisée en ce qu'**en tant qu'additif, on utilise du chlorure ou carbonate de magnésium en une quantité de 1 à 5 g/l.

8. Composition selon la revendication 1, 3 et 4, **caractérisée en ce qu'**en tant qu'additif, on utilise du sel de mer en une quantité de 1 à 320 g/l.

9. Composition selon les revendications 1 à 4, **caractérisée en ce qu'**en tant qu'additif, on utilise un mélange desdits composés en une quantité de 2 à 320 g/l.

10. Composition selon les revendications 1 à 9, **caractérisée en ce que** ladite composition contient de plus des microéléments sous la forme de chlorures ou carbonates de cuivre, molybdène, manganèse, fer, cobalt, nickel en une quantité de 1 à 4 g/l.

11. Composition selon les revendications 1 et 2, **caractérisée en ce que** les impulsions électriques sont préconverties en impulsions magnétiques, électromagnétiques, lumineuses ou acoustiques.
